# EUROPEAN PATENT APPLICATION

(11) **EP 1 568 381 A1**
(43) Date of publication of application: **31.08.2005**
(21) Application number: 03754063.0
(22) Date of filing: 09.10.2003
(51) Int. Cl.: A61K 45/06, A61K 31/567, A61P 35/00, A61P 43/00

(54) **REMEDY FOR HORMONE-DEPENDENT CANCER**

(30) Priority: 09.10.2002 JP 2002295898
(71) Applicant: KYOWA HAKKO KOGYO CO., LTD., Tokyo 100-8185 (JP)
(72) Inventor: SHIOTSU, Yukimasa, Pharmaceutical Research Center, Sunto-gun, Shizuoka 411-8731 (JP); ISHIDA, Hiroyuki, Pharmaceutical Research Center, Sunto-gun, Shizuoka 411-8731 (JP); MURAKATA, Chikara, Head Office, Kyowa Hakko Co.,Lt, Tokyo 100-8185 (JP); KUSAKA, Hideaki, Pharmaceutical Research Center, Sunto-gun, Shizuoka 411-8731 (JP); AKINAGA, Shiro, Head Office, Kyowa Hakko Kogyo CO, Tokyo 100-8185 (JP)
(74) Representative: Vossius & Partner
(86) International application number: PCT/JP2003/012986
(87) International publication number: WO 2004/035089

(57) **Abstract**

A therapeutic agent for a hormone-dependent cancer, which comprises (a) a steroid-sulfatase inhibitor and (b) an agent for hormone therapy and/or an agent for chemotherapy, which may be administered together or separately at an interval, is provided. A method for treating a hormone-dependent cancer, which comprises administering (a) a steroid-sulfatase inhibitor and (b) an agent for hormone therapy and/or an agent for chemotherapy together or separately at an interval, is also provided. A steroid-sulfatase inhibitor which is used in combination with an agent for hormone therapy and/or an agent for chemotherapy, and which is administered together therewith or separately therefrom at an interval, is also provided. A kit for treating a hormone-dependent cancer, which comprises a first component comprising (a) a steroid-sulfatase inhibitor and a second component comprising (b) an agent for hormone therapy and/or an agent for chemotherapy, is also provided. A pharmaceutical composition, which comprises (a) a steroid-sulfatase inhibitor and (b) an agent for hormone therapy and/or an agent for chemotherapy, is also provided. Further, use of (a) a steroid-sulfatase inhibitor and (b) an agent for hormone therapy and/or an agent for chemotherapy for the manufacture of a therapeutic agent for a hormone-dependent cancer is provided.

## Description

### Technical Field

The present invention relates to a therapeutic agent for a hormone-dependent cancer, comprising a steroid-sulfatase inhibitor.

### Background Art

Among cancers, there are those wherein proliferation thereof is promoted by hormone(s) (hormone-dependent cancers). Such hormone-dependent cancers include breast cancer, ovarian cancer, endometrial cancer, prostatic cancer, and thyroid cancer.

Nowadays, the hormone-dependent cancers are treated by surgical removal of an organ that secretes a particular hormone (e.g. surgical removal of the ovary), by the administration of an inhibitor that reduces hormone activities in order to suppress the proliferation of the hormone-dependent cancer cells (e.g. hormone therapy and chemotherapy), or the like. In some cases, these therapies may be performed in combination.

Examples of the agents for hormone therapy include antiestrogen agents, aromatase inhibitors, antiandrogen agents, preparations comprising progesterone, and preparations comprising an luteinizing hormone-releasing hormone (LH-RH) agonist.

On the other hand, steroid sulfatase is a hydrolase that converts estrone sulfate, i.e. inactive estrogen, to estrone, i.e. active estrogen, and that converts androstenediol sulfate, i.e. inactive androgen, to androstenediol, i.e. active androgen. Thus, steroid sulfatase is involved in the proliferation of mammary gland epithelial cells, hormone-dependent cancer cells or tumor cells.

A high estrogen level in breast cancer is considered to be caused by the hydrolysis of estrone sulfate to estrone by steroid sulfatase (estrone sulfatase). Therefore, steroid-sulfatase inhibitors are considered to be effective therapeutic agents for the treatment of estrogen-dependent breast cancer (a hormone-dependent cancer), and further to be effective for preventing or treating other diseases in which estrones are considered to be involved, e.g. endometrial cancer, ovarian cancer, endometriosis, and adenomyosis uteri. Further, since steroid sulfatase is also involved in the biosynthetic process of androgen, it is considered to be effective for preventing or treating diseases in which androgens are considered to be involved, e.g. prostatic cancer.

It has been reported that estrone-3-sulfamate (EMATE) is a typical inhibitor of steroid sulfatase (See, e.g. U.S. Patent No. 5,616,574; International Journal of Cancer, 1995, 63: 106-111). However, it has been shown that EMATE is not effective in the treatment of estrone-dependent diseases because of its estrogen-like activity (See, e.g. Cancer Research, 1996, 56: 4950-4955).

So far, a large number of steroid-sulfatase inhibitors have been found (See, e.g. U.S. Patent No. 5,830,886; WO98/11124; WO98/32763; Expert Opinion on Therapeutic Patents, 1999, 9: 1083).

Such inhibitors include tyramine derivatives (See, e.g. U.S. Patent No. 5,567,831; Cancer Research, 1997, 57: 702-707; The Journal of Steroid Biochemistry and Molecular Biology, 1996, 59: 41-48; The Journal of Steroid Biochemistry and Molecular Biology, 1999, 68: 31-40; The Journal of Steroid Biochemistry and Molecular Biology, 1999, 69: 227-238), cinnamic acid derivatives (See, e.g. U.S. Patent No. 6,011,024), and diethylstilbestrol derivatives (See, e.g. The Journal of Steroid Biochemistry and Molecular Biology, 1999, 69: 227-238). Recently, other steroid-sulfatase inhibitors have been disclosed (See, e.g. WO01/04086; WO01/02349).

Furthermore, estrone-3-methylthiophosphonate, estrone-3-methylphosphonate, estrone-3-phenylphosphonothioate, estrone-3-phenylphosphonate (See, e.g. U.S. Patent No. 5,604,215; Cancer Research, 1993, 53: 298-303; Bioorganic & Medicinal Chemistry Letters, 1993, 3: 313-318), and 3-monoalkylthiophosphate derivatives (See, e.g. WO91/13083) have been disclosed as steroid-sulfatase inhibitors.

In addition to the above, other steroid-sulfatase inhibitors have been disclosed (See, e.g. WO93/05064; WO97/30041; WO99/33858; WO99/52890; WO01/36398; WO00/43408).

### Disclosure of the Invention

An object of the present invention is to provide a therapeutic agent for a hormone-dependent cancer, which comprises a steroid-sulfatase inhibitor, and an agent for hormone therapy and/or an agent for chemotherapy, and the like.

The present invention relates to the following (1) to (36):
(1) A therapeutic agent for a hormone-dependent cancer, which comprises (a) a steroid-sulfatase inhibitor and (b) an agent for hormone therapy and/or an agent for chemotherapy, which may be administered together or separately at an interval.
(2) A method for treating a hormone-dependent cancer, which comprises administering (a) a steroid-sulfatase inhibitor and (b) an agent for hormone therapy and/or an agent for chemotherapy together or separately at an interval.
(3) A steroid-sulfatase inhibitor which is used in combination with an agent for hormone therapy and/or an agent for chemotherapy, and which is administered together therewith or separately therefrom at an interval.
(4) A kit for treating a hormone-dependent cancer, which comprises a first component comprising (a) a steroid-sulfatase inhibitor and a second component comprising (b) an agent for hormone therapy and/or an agent for chemotherapy.
(5) A pharmaceutical composition, which comprises (a) a steroid-sulfatase inhibitor and (b) an agent for hormone therapy and/or an agent for chemotherapy.
(6) Use of (a) a steroid-sulfatase inhibitor and (b) an agent for hormone therapy and/or an agent for chemotherapy for the manufacture of a therapeutic agent for a hormone-dependent cancer.
(7) The therapeutic agent for a hormone-dependent cancer according to (1), wherein the steroid-sulfatase inhibitor is a composition comprising, as an active ingredient, a compound represented by Formula (I) or a pharmaceutically acceptable salt thereof: 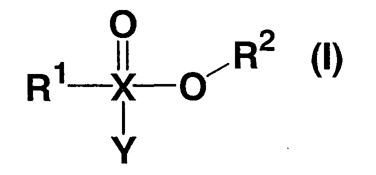 [wherein X represents a phosphorus atom or a sulfur atom, and when X is a phosphorus atom, Y is hydroxy, and when X is a sulfur atom, Y is oxo; R¹ represents a hydrogen atom, substituted or unsubstituted lower alkyl, substituted or unsubstituted cycloalkyl, substituted or unsubstituted lower alkenyl, substituted or unsubstituted aryl, or -NR³R⁴ (wherein R³ and R⁴ may be the same or different and each represents a hydrogen atom, substituted or unsubstituted lower alkyl, substituted or unsubstituted cycloalkyl, substituted or unsubstituted lower alkenyl, or substituted or unsubstituted aryl, or R³ and R⁴ are combined together with the adjacent nitrogen atom thereto to form a substituted or unsubstituted heterocyclic group); and -O-R² represents a monocyclic alcohol residue or a polycyclic alcohol residue].
(8) The method for treating a hormone-dependent cancer according to (2), wherein the steroid-sulfatase inhibitor is a composition comprising, as an active ingredient, the compound represented by Formula (I) described in (7) or a pharmaceutically acceptable salt thereof.
(9) The steroid-sulfatase inhibitor according to (3), wherein the steroid-sulfatase inhibitor is a composition comprising, as an active ingredient, the compound represented by Formula (I) described in (7) or a pharmaceutically acceptable salt thereof.
(10) The kit for treating according to (4), wherein the steroid-sulfatase inhibitor is a composition comprising, as an active ingredient, the compound represented by Formula (I) described in (7) or a pharmaceutically acceptable salt thereof.
(11) The pharmaceutical composition according to (5), wherein the steroid-sulfatase inhibitor is a composition comprising, as an active ingredient, the compound represented by Formula (I) described in (7) or a pharmaceutically acceptable salt thereof.
(12) The use of (a) a steroid-sulfatase inhibitor and (b) an agent for hormone therapy and/or an agent for chemotherapy according to (6), wherein the steroid-sulfatase inhibitor is a composition comprising, as an active ingredient, the compound represented by Formula (I) described in (7) or a pharmaceutically acceptable salt thereof.
(13) The therapeutic agent for a hormone-dependent cancer according to (1), wherein the steroid-sulfatase inhibitor is a composition comprising, as an active ingredient, a compound represented by Formula (IA) or a pharmaceutically acceptable salt thereof: 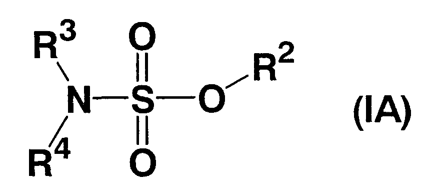 (wherein -O-R², R³, and R⁴ have the same meanings as defined above, respectively).
(14) The method for treating a hormone-dependent cancer according to (2), wherein the steroid-sulfatase inhibitor is a composition comprising, as an active ingredient, the compound represented by Formula (IA) described in (13) or a pharmaceutically acceptable salt thereof.
(15) The steroid-sulfatase inhibitor according to (3), wherein the steroid-sulfatase inhibitor is a composition comprising, as an active ingredient, the compound represented by Formula (IA) described in (13) or a pharmaceutically acceptable salt thereof.
(16) The kit for treating according to (4), wherein the steroid-sulfatase inhibitor is a composition comprising, as an active ingredient, the compound represented by Formula (IA) described in (13) or a pharmaceutically acceptable salt thereof.
(17) The pharmaceutical composition according to (5), wherein the steroid-sulfatase inhibitor is a composition comprising, as an active ingredient, the compound represented by Formula (IA) described in (13) or a pharmaceutically acceptable salt thereof.
(18) The use of (a) a steroid-sulfatase inhibitor and (b) an agent for hormone therapy and/or an agent for chemotherapy according to (6), wherein the steroid-sulfatase inhibitor is a composition comprising, as an active ingredient, the compound represented by Formula (IA) described in (13) or a pharmaceutically acceptable salt thereof.
(19) The therapeutic agent for a hormone-dependent cancer according to (1), wherein the steroid-sulfatase inhibitor is a composition comprising, as an active ingredient, a compound represented by Formula (IB) or a pharmaceutically acceptable salt thereof: 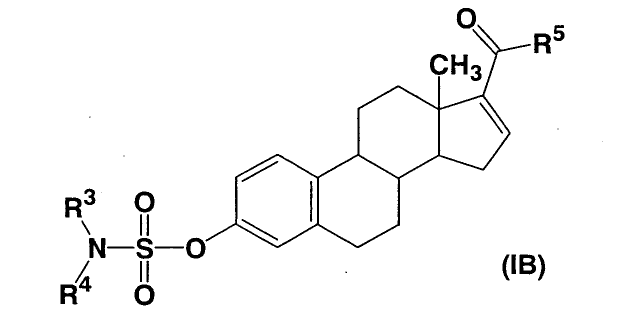 [wherein R³ and R⁴ have the same meanings as defined above, respectively; R⁵ represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, -NR⁶R⁷ (wherein R⁶ and R⁷ may be the same or different and each represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), -OR⁸ (wherein R⁸ represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), or -SR^{8A} (wherein R^{8A} has the same meaning as R⁸ defined above)].
(20) The method for treating a hormone-dependent cancer according to (2), wherein the steroid-sulfatase inhibitor is a composition comprising, as an active ingredient, the compound represented by Formula (IB) described in (19) or a pharmaceutically acceptable salt thereof.
(21) The steroid-sulfatase inhibitor according to (3), wherein the steroid-sulfatase inhibitor is a composition comprising, as an active ingredient, the compound represented by Formula (IB) described in (19) or a pharmaceutically acceptable salt thereof.
(22) The kit for treating according to (4), wherein the steroid-sulfatase inhibitor is a composition comprising, as an active ingredient, the compound represented by Formula (IB) described in (19) or a pharmaceutically acceptable salt thereof.
(23) The pharmaceutical composition according to (5), wherein the steroid-sulfatase inhibitor is a composition comprising, as an active ingredient, the compound represented by Formula (IB) described in (19) or a pharmaceutically acceptable salt thereof.
(24) The use of (a) a steroid-sulfatase inhibitor and (b) an agent for hormone therapy and/or an agent, for chemotherapy according to (6), wherein the steroid-sulfatase inhibitor is a composition comprising, as an active ingredient, the compound represented by Formula (IB) described in (19) or a pharmaceutically acceptable salt thereof.
(25) The therapeutic agent for a hormone-dependent cancer according to (1), (7), (13), or (19), wherein the agent for hormone therapy is one or more selected from the group consisting of an antiestrogen agent, an aromatase inhibitor, an antiandrogen agent, a preparation comprising progesterone, and a preparation comprising a luteinizing hormone-releasing hormone (LH-RH) agonist.
(26) The method for treating a hormone-dependent cancer according to (2), (8), (14), or (20), wherein the agent for hormone therapy is one or more selected from the group consisting of an antiestrogen agent, an aromatase inhibitor, an antiandrogen agent, a preparation comprising progesterone, and a preparation comprising a LH-RH agonist.
(27) The steroid-sulfatase inhibitor according to (3), (9), (15), or (21), wherein the agent for hormone therapy is one or more selected from the group consisting of an antiestrogen agent, an aromatase inhibitor, an antiandrogen agent, a preparation comprising progesterone, and a preparation comprising a LH-RH agonist.
(28) The kit for treating according to (4), (10), (16), or (22), wherein the agent for hormone therapy is one or more selected from the group consisting of an antiestrogen agent, an aromatase inhibitor, an antiandrogen agent, a preparation comprising progesterone, and a preparation comprising a LH-RH agonist.
(29) The pharmaceutical composition according to (5), (11), (17), or (23), wherein the agent for hormone therapy is one or more selected from the group consisting of an antiestrogen agent, an aromatase inhibitor, an antiandrogen agent, a preparation comprising progesterone, and a preparation comprising a LH-RH agonist.
(30) The use of (a) a steroid-sulfatase inhibitor and (b) an agent for hormone therapy and/or an agent for chemotherapy according to (6), (12), (18), or (24), wherein the agent for hormone therapy is one or more selected from the group consisting of an antiestrogen agent, an aromatase inhibitor, an antiandrogen agent, a preparation comprising progesterone, and a preparation comprising a LH-RH agonist.
(31) The therapeutic agent for a hormone-dependent cancer according to (1), (7), (13), or (19), wherein the agent for hormone therapy is an antiestrogen agent and/or an aromatase inhibitor.
(32) The method for treating a hormone-dependent cancer according to (2), (8), (14), or (20), wherein the agent for hormone therapy is an antiestrogen agent and/or an aromatase inhibitor.
(33) The steroid-sulfatase inhibitor according to (3), (9), (15), or (21), wherein the agent for hormone therapy is an antiestrogen agent and/or an aromatase inhibitor.
(34) The kit for treating according to (4), (10), (16), or (22), wherein the agent for hormone therapy is an antiestrogen agent and/or an aromatase inhibitor.
(35) The pharmaceutical composition according to (5), (11), (17), or (23), wherein the agent for hormone therapy is an antiestrogen agent and/or an aromatase inhibitor.
(36) The use of (a) a steroid-sulfatase inhibitor and (b) an agent for hormone therapy and/or an agent for chemotherapy according to (6), (12), (18), or (24), wherein the agent for hormone therapy is an antiestrogen agent and/or an aromatase inhibitor.

Any hormone-dependent cancer or tumor, in which cancer cells or tumor cells are stimulated to proliferate by a hormone, can be exemplified as the hormone-dependent cancer treated in the present invention. Such hormone-dependent cancers include breast cancer, ovarian cancer, endometrial cancer, prostatic cancer, and thyroid cancer.

Any steroid-sulfatase inhibitor, which can inhibit the steroid sulfatase activity, can be used as the steroid-sulfatase inhibitor. Examples of such steroid-sulfatase inhibitors include a composition comprising, as an active ingredient, a sulfonate ester, a phosphonate ester, a sulfamate, or a thiophosphate of a monocyclic alcohol or a polycyclic alcohol, or the like or a pharmaceutically acceptable salt thereof.

Specifically, the composition which comprises, as an active ingredient, a compound represented by Formula (I) or a pharmaceutically acceptable salt thereof and the like are exemplified: 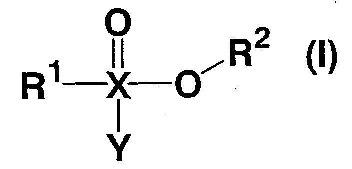 [wherein X represents a phosphorus atom or a sulfur atom, and when X is a phosphorus atom, Y is hydroxy, and when X is a sulfur atom, Y is oxo; R¹ represents a hydrogen atom, substituted or unsubstituted lower alkyl, substituted or unsubstituted cycloalkyl, substituted or unsubstituted lower alkenyl, substituted or unsubstituted aryl, or -NR³R⁴ (wherein R³ and R⁴ may be the same or different and each represents a hydrogen atom, substituted or unsubstituted lower alkyl, substituted or unsubstituted cycloalkyl, substituted or unsubstituted lower alkenyl, or substituted or unsubstituted aryl, or R³ and R⁴ are combined together with an adjacent nitrogen atom thereto to form a substituted or unsubstituted heterocyclic group); and -O-R² represents a monocyclic alcohol residue or a polycyclic alcohol residue].

Among them, the composition which comprises, as an active ingredient, a compound represented by Formula (IA) or a pharmaceutically acceptable salt thereof and the like are preferred: 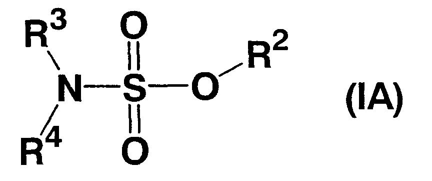 (wherein -O-R², R³, and R⁴ have the same meanings as defined above, respectively). A composition which comprises, as an active ingredient, a compound represented by Formula (IB) or a pharmaceutically acceptable salt thereof and the like are more preferred: 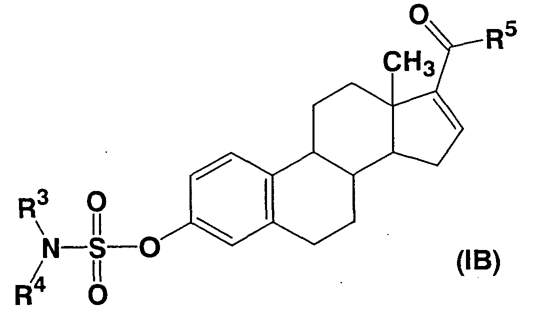 [wherein R³ and R⁴ have the same meanings as defined above, respectively; R⁵ represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, -NR⁶R⁷ (wherein R⁶ and R⁷ may be the same or different and each represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), -OR⁸ (wherein R⁸ represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), or -SR^{8A} (wherein R^{8A} has the same meaning as R⁸ defined above)].

A compound represented by Formula (I) is referred to as Compound (I) hereinafter. Compounds represented by other Formula numbers are also referred to in the same manner.

In the definition of each group in Formulae (I), (IA), and (IB), (i) the monocyclic and polycyclic alcohols constituting monocyclic or polycyclic alcohol residues include any monocyclic and polycyclic alcohol. For example, sulfate compounds (the hydroxyl group is replaced by a sulfate group) corresponding to the alcohols that can be substrates of the steroid sulfatase are preferred. Among them, sulfate compounds having a Km value of less than 50 µmol/L during incubation at pH 7.4 and 37°C with an enzyme having a steroid sulfatase activity are more preferred.

Examples of the monocyclic alcohol include a substituted or unsubstituted heterocycle having hydroxy as one of substituents thereof [the heterocycle corresponds to a compound formed by adding one hydrogen atom to a heterocyclic group (x) described later; and substituents other than hydroxy of the substituted heterocycle correspond to substituents of the substituted heterocyclic group (xii) described later], and a substituted or unsubstituted phenol [substituents of the substituted phenol correspond to substituents of substituted heterocyclic group (xii) described later]. Specific examples include tyramine amide derivatives, hydroxycinnamic acid derivatives, and the like.

Examples of the polycyclic alcohol include substituted or unsubstituted fused rings. Examples of the fused ring include di- to penta-cyclic fused rings having 6 to 60 carbon atoms, preferably 6 to 30 carbon atoms and formed by condensing 3- to 8-membered rings having a hydroxyl group as one of the substituents. Each ring may be saturated or unsaturated and may include an element such as a nitrogen atom, an oxygen atom, and a sulfur atom. Specific examples include substituted or unsubstituted sterols; tetrahydronaphthol derivatives; coumarin, chroman, or isoflavone derivatives each having a hydroxyl group as one of substituents; and 4-hydroxytamoxifen derivatives. Substituents of the substituted fused ring and the substituted sterol correspond to substituents of substituted sterol (iii) described later.

(ii) Examples of the sterol include 3-sterol such as estrone, estradiol, estriol, and dehydroepiandrosterone.

(iii) Substituents of the substituted sterol described here may be the same or different. The number of the substituents may be 1 to 3, and examples of the substituents include halogen, nitro, cyano, azide, substituted or unsubstituted lower alkyl, substituted or unsubstituted cycloalkyl, substituted or unsubstituted lower alkenyl, substituted or unsubstituted lower alkynyl, substituted or unsubstituted aryl, substituted or unsubstituted heterocyclic group, -C(=X¹)R⁵ (wherein X¹ represents an oxygen atom or a sulfur atom, R⁵ has the same meaning as defined above), -NR⁹R¹⁰ {wherein R⁹ and R¹⁰ may be the same or different and each represents a hydrogen atom, substituted or unsubstituted lower alkyl, substituted or unsubstituted cycloalkyl, substituted or unsubstituted lower alkenyl, substituted or unsubstituted aryl, a substituted or unsubstituted heterocyclic group, -C(=X²)R¹¹ (wherein X² and R¹¹ have the same meanings as X¹ and R⁵ defined above, respectively), or -SO₂R¹² [wherein R¹² represents substituted or unsubstituted lower alkyl, substituted or unsubstituted cycloalkyl, substituted or unsubstituted lower alkenyl, substituted or unsubstituted aryl, a substituted or unsubstituted heterocyclic group, -NR¹³R¹⁴ (wherein R¹³ and R¹⁴ have the same meanings as R⁶ and R⁷ defined above, respectively), or -OR¹⁵ (wherein R¹⁵ has the same meaning as R⁸ defined above)]}, -OR¹⁶ [wherein R¹⁶ represents a hydrogen atom, substituted or unsubstituted lower alkyl, substituted or unsubstituted cycloalkyl, substituted or unsubstituted lower alkenyl, substituted or unsubstituted aryl, a substituted or unsubstituted heterocyclic group, or -SO₂R¹⁷ (wherein R¹⁷ has the same meaning as R¹² defined above)], -S(O)ₘR¹⁸ (wherein m represents 0 or 1, R¹⁸ represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), or -SO₂R¹⁹ (wherein R¹⁹ has the same meaning as R¹² described above).

The halogen, lower alkyl, cycloalkyl, lower alkenyl, lower alkynyl, aryl, and heterocyclic group mentioned here, have the same meanings as the halogen (ix), lower alkyl (iv), cycloalkyl (vii), lower alkenyl (v), lower alkynyl (vi), aryl (viii), and heterocyclic group (x) defined later, respectively. Substituents of the substituted lower alkyl, substituted lower alkenyl, and substituted lower alkynyl have the same meanings as substituents of the substituted lower alkyl (xiii) defined later, respectively, and substituents of the substituted cycloalkyl, substituted aryl, and substituted heterocyclic group have the same meanings as substituents of the substituted cycloalkyl (xvi) defined later, respectively.

Specifically, examples of the substituted sterol include substituted sterol having hydroxy at 3-position, for example, substituted estrone such as 2-hydroxyestrone, 2-methoxyestrone, 4-hydroxyestrone, 6α-hydroxyestrone, 1α-hydroxyestrone, 15α-hydroxyestrone, and 15β-hydroxyestrone; substituted estradiol such as 2-hydroxy-17β-estradiol, 2-methoxy-17β-estradiol, 4-hydroxy-17β-estradiol, 6α-hydroxy-17β-estradiol, 7α-hydroxy-17β-estradiol, 16α-hydroxy-17α-estradiol, 16β-hydroxy-17α-estradiol, 16β-hydroxy-17β-estradiol, 17α-estradiol, 17β-estradiol, and 17α-ethynyl-17β-estradiol; substituted estriol such as 2-hydroxyestriol, 2-methoxyestriol, 4-hydroxyestriol, 6α-hydroxyestriol, and 7α-hydroxyestriol; and substituted dehydroepiandrosterone such as 6α-hydroxydehydroepiandrosterone, 7α-hydroxydehydroepiandrosterone, 16α-hydroxydehydroepiandrosterone, and 16β-hydroxydehydroepiandrosterone. These substituted sterol may further have the above-mentioned substituents.

(iv) Examples of the lower alkyl include linear or branched alkyl having 1 to 20 carbon atoms, e.g. methyl, ethyl, propyl, isopropyl, butyl, isobutyl, sec-butyl, tert-butyl, pentyl, isopentyl, neopentyl, hexyl, heptyl, octyl, isooctyl, decyl, undecyl, dodecyl, tridecyl, tetradecyl, pentadecyl, and eicocyl.

(v) Examples of the lower alkenyl include linear or branched alkenyl having 2 to 8 carbon atoms, e.g. vinyl, allyl, 1-propenyl, butenyl, pentenyl, hexenyl, heptenyl, and octenyl.

(vi) Examples of the lower alkynyl include linear or branched alkynyl having 2 to 8 carbon atoms, e.g. ethynyl, propynyl, butynyl, pentynyl, hexynyl, heptynyl, and octynyl.

(vii) Examples of the cycloalkyl include cycloalkyl having 3 to 8 carbon atoms, e.g. cyclopropyl, cyclobutyl, cyclopentyl, cyclohexyl, cycloheptyl, and cyclooctyl.

(viii) Examples of the aryl include aryl having 6 to 14 carbon atoms, e.g. phenyl, naphthyl, and anthryl.

(ix) Examples of the halogen include fluorine, chlorine, bromine, and iodine atoms.

(x) Examples of the heterocyclic group include an aliphatic heterocyclic group and an aromatic heterocyclic group.

Examples of the aliphatic heterocyclic group include a 5- or 6-membered monocyclic group containing at least one atom selected from a nitrogen atom, an oxygen atom, and a sulfur atom, and a bicyclic or tricyclic fused ring which is formed by condensation 3- to 8-membered rings and which contains at least one atom selected from a nitrogen atom, an oxygen atom, and a sulfur atom. Specific examples include tetrahydropyranyl, pyranyl, tetrahydrofuranyl, pyrrolidinyl, piperidino, piperidyl, perhydroazepinyl, perhydroazocinyl, morpholino, morpholinyl, thiomorpholino, thiomorpholinyl, piperazinyl, homopiperazinyl, oxazolinyl, dioxolanyl, imidazolidinyl, imidazolinyl, pyrazolidinyl, indolinyl, 1-oxo-1,3-dihydroisoindolyl, 1,1-dioxo-2,3-dihydrobenz[d]isothiazolyl, 2-pyrrolinyl, 2-pyrrolidonyl, 3-pyrrolidonyl, 2-piperidonyl, 3-piperidonyl, 4-piperidonyl, perhydro-2-azepinonyl, perhydro-3-azepinonyl, perhydro-4-azepinonyl, 2-thiazolidonyl, 4-thiazolidonyl, 2-oxazolidonyl, 4-oxazolidonyl, succinimide, glutarimide, hydantoinyl, thiazolidinedionyl, oxazolidinedionyl, and the like.

Examples of the aromatic heterocyclic group include a 5- or 6-membered monocyclic group containing at least one atom selected from an nitrogen atom, an oxygen atom, and a sulfur atoms, and bicyclic or tricyclic fused ring which is formed by condensation of 3- to 8-membered rings and contains at least one atom selected from a nitrogen atom, an oxygen atom, and a sulfur atom. Specific examples include furyl, thienyl, pyrrolyl, imidazolyl, pyrazolyl, triazolyl, tetrazolyl, oxazolyl, isoxazolyl, thiazolyl, isothiazolyl, 1,2,3-oxadiazolyl, 1,2,4-oxadiazolyl, 1,3,4-oxadiazolyl, 1,2,3-thiadiazolyl, 1,2,4-thiadiazolyl, 1,3,4-thiadiazolyl, furazanyl, pyridyl, pyrimidinyl, pyrazinyl, pyridazinyl, quinolyl, isoquinolyl, quinazolinyl, phthalazinyl, purinyl, indolyl, isoindolyl, 2-pyridonyl, 4-pyridonyl, uracilyl, benzofuryl, benzothienyl, benzimidazolyl, benzoxazolyl, benzisoxazolyl, benzothiazolyl, benzisothiazolyl, 1,3-dioxo-1,3-dihydroisoindolyl, 1,1,3-trioxo-2,3-dihydrobenz[d]isothiazolyl, maleimido, phthalimido, and the like.

(xi) Examples of the heterocyclic group formed together with the adjacent nitrogen atom may contain an oxygen atom, a sulfur atom, or a nitrogen atom other than the adjacent nitrogen atom. Specific examples include pyrrolidinyl, thiazolidinyl, oxazolidinyl, piperidino, homopiperidino, piperazinyl, homopiperazinyl, pyrazolidinyl, morpholino, thiomorpholino, tetrahydroquinolyl, tetrahydroisoquinolyl, octahydroquinolyl, benzimidazolyl, indazolyl, indolyl, isoindolyl, purinyl, dihydroindolyl, pyrrolyl, pyrazolyl, triazolyl, tetrazolinyl, imidazolyl, and the like.

(xii) Substituents of the substituted heterocyclic group may be the same or different. The number of the substituents is 1 to 3, and examples of the substituents include halogen, nitro, cyano, azido, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, -C(=X¹)R⁵ (wherein X¹ and R⁵ have the same meanings as defined above, respectively), -NR⁹R¹⁰ {wherein R⁹ and R¹⁰ have the same meanings as defined above, respectively), -OR¹⁶ (wherein R¹⁶ has the same meaning as defined above), -S(O)ₘR¹⁸ (wherein m and R¹⁸ have the same meanings as defined above, respectively), and -SO₂R¹⁹ (wherein R¹⁹ has the same meaning as defined above).

The halogen, lower alkyl, cycloalkyl, lower alkenyl, lower alkynyl, aryl, and heterocyclic group mentioned here, have the same meanings as the halogen (ix), lower alkyl (iv), cycloalkyl (vii), lower alkenyl (v), lower alkynyl (vi), aryl (viii), and heterocyclic group (x) defined above, respectively. The substituents of the substituted lower alkyl, substituted lower alkenyl, and substituted lower alkynyl have the same meanings as substituents of the substituted lower alkyl (xiii) defined later, respectively, and the substituents of the substituted cycloalkyl, substituted aryl, and substituted heterocyclic group have the same meanings as substituents of the substituted cycloalkyl (xvi) defined later, respectively.

(xiii) The substituents of the substituted lower alkyl, substituted lower alkenyl, and substituted lower alkynyl may be the same or different. The number of substituents is 1 to 3, and examples of the substituents include halogen, nitro, cyano, azido, lower alkenyl, lower alkadienyl, lower alkatrienyl, lower alkynyl, (lower alkoxy)lower alkoxy, substituted or unsubstituted cycloalkyl, substituted or unsubstituted aryl, a substituted or unsubstituted heterocyclic group, -C(=X^{1A})R^{5A} [wherein X^{1A} has the same meaning as X¹ defined above, and R^{5A} represents a hydrogen atom, lower alkyl, substituted or unsubstituted cycloalkyl, lower alkenyl, lower alkynyl, substituted or unsubstituted aryl, a substituted or unsubstituted heterocyclic group, substituted or unsubstituted aralkyl, substituted or unsubstituted heteroarylalkyl, -NR^{6A}R^{7A} (wherein R^{6A} and R^{7A} may be the same or different and each represents a hydrogen atom, lower alkyl, substituted or unsubstituted cycloalkyl, lower alkenyl, substituted or unsubstituted aryl, a substituted or unsubstituted heterocyclic group, substituted or unsubstituted aralkyl, or substituted or unsubstituted heteroarylalkyl), -OR^{BA} (wherein R^{8A} represents a hydrogen atom, lower alkyl, substituted or unsubstituted cycloalkyl, lower alkenyl, substituted or unsubstituted aryl, a substituted or unsubstituted heterocyclic group, substituted or unsubstituted aralkyl, or substituted or unsubstituted heteroarylalkyl), or -SR^{8Aa} (wherein R^{8Aa} has the same meaning as R^{8A} defined above)], -NR^{9A}R^{10A} {wherein R^{9A} and R^{10A} may be the same or different and each represents a hydrogen atom, lower alkyl, substituted or unsubstituted cycloalkyl, lower alkenyl, substituted or unsubstituted aryl, a substituted or unsubstituted heterocyclic group, substituted or unsubstituted aralkyl, substituted or unsubstituted heteroarylalkyl, -C(=X^{2A})R^{11A} (wherein X^{2A} and R^{11A} have the same meanings as X^{1A} and R^{4A} defined above, respectively), or -SO₂R^{12A} [wherein R^{12A} represents lower alkyl, substituted or unsubstituted cycloalkyl, lower alkenyl, substituted or unsubstituted aryl, a substituted or unsubstituted heterocyclic group, substituted or unsubstituted aralkyl, substituted or unsubstituted heteroarylalkyl, -NR^{13A}R^{14A} (wherein R^{13A} and R^{14A} have the same meanings as R^{6A} and R^{7A} defined above, respectively), or -OR^{15A} (wherein R^{15A} has the same meaning as R^{8A} defined above)]}, -OR^{16A} [wherein R^{16A} represents a hydrogen atom, lower alkyl, substituted or unsubstituted cycloalkyl, lower alkenyl, substituted or unsubstituted aryl, a substituted or unsubstituted heterocyclic group, substituted or unsubstituted aralkyl, substituted or unsubstituted heteroarylalkyl, or -SO₂R^{17A} (wherein R^{17A} has the same meaning as R^{12A} defined above)], -S(O)ₘₐR^{18A} (wherein ma represents 0 or 1, R^{18A} represents a hydrogen atom, lower alkyl, substituted or unsubstituted cycloalkyl, lower alkenyl, substituted or unsubstituted aryl, a substituted or unsubstituted heterocyclic group, substituted or unsubstituted aralkyl, or substituted or unsubstituted heteroarylalkyl), or -SO₂R^{19A} (wherein R^{19A} has the same meaning as R^{12A} defined above).

The halogen, lower alkyl, lower alkenyl, lower alkynyl, cycloalkyl, aryl, and heterocyclic group mentioned here, have the same meanings as the halogen (ix), lower alkyl (iv), lower alkenyl (v), lower alkynyl (vi), cycloalkyl (vii), aryl (viii), and heterocyclic group (x) described above, respectively. Examples of the lower alkadienyl (xiv) include alkadienyl having 4 to 8 carbon atoms, e.g. 1,3-butadienyl, 1,3-pentadienyl, 1,3-hexadienyl, 2,4-hexadienyl, and 1,3-octadienyl. Examples of the lower alkatrienyl (xv) include alkatrienyl having 6 to 8 carbon atoms, e.g. 1,3,5-hexatrienyl and 1,3,5-octatrienyl. A lower alkyl moiety of the (lower alkoxy)lower alkoxy has the same meaning as the lower alkyl (iv) defined above. The alkylene moieties of the (lower alkoxy)lower alkoxy, aralkyl, and heteroarylalkyl have the same meanings as the group formed by removing one hydrogen atom from lower alkyl (iv) defined above. Aryl moiety of the aralkyl group has the same meaning as the aryl (viii) defined above, and heteroaryl moiety of the heteroarylalkyl has the same meaning as the aromatic heterocyclic group in the heterocyclic group (x) defined above.

Substituents of the substituted cycloalkyl, substituted aryl, substituted heterocyclic, substituted aralkyl, and substituted heteroarylalkyl mentioned here, have the same meanings as the substituents of the substituted cycloalkyl (xvi) defined later, respectively.

(xiv) The substituents of the substituted cycloalkyl, substituted aryl, and substituted heterocyclic group formed together with the adjacent nitrogen atom may be the same or different. The number of the substituents is 1 to 3, and examples of the substituents include lower alkyl, halogen, nitro, cyano, azido, lower alkenyl, lower alkadienyl, lower alkatrienyl, lower alkynyl, (lower alkoxy)lower alkoxy, cycloalkyl, aryl, 4-sulfamoyloxybenzyl, a heterocyclic group, -C(=X^{1B})R^{5B} [wherein X^{1B} has the same meaning as X¹ defined above, and R^{5B} represents a hydrogen atom, lower alkyl, cycloalkyl, lower alkenyl, lower alkynyl, aryl, a heterocyclic group, -NR^{6B}R^{7B} (wherein R^{6B} and R^{7B} may be the same or different and each represents a hydrogen atom, lower alkyl, cycloalkyl, lower alkenyl, aryl, or a heterocyclic group), -OR^{8Ba} (wherein R^{8B} represents a hydrogen atom, lower alkyl, cycloalkyl, lower alkenyl, aryl, or a heterocyclic group), or -SR^{8Ba} (wherein R^{8Ba} has the same meaning as R^{8B} defined above)], -NR^{9B}R^{10B} {wherein R^{9B} and R^{10B} may be the same or different and each represents a hydrogen atom, lower alkyl, cycloalkyl, lower alkenyl, aryl, a heterocyclic group, -C(=X^{2B})R^{11B} (wherein X^{2B} and R^{11B} have the same meanings as X^{1B} and R^{5B} defined above, respectively), or -SO₂R^{12B} [wherein R^{12B} represents lower alkyl, cycloalkyl, lower alkenyl, aryl, a heterocyclic group, -NR^{13B}R^{14B} (wherein R^{13B} and R^{14B} have the same meanings as R^{6B} and R^{7B} defined above, respectively), or -OR^{15B} (wherein R^{15B} has the same meaning as R^{8B} defined above)]}, -OR^{16B} [wherein R^{16B} represents a hydrogen atom, lower alkyl, cycloalkyl, lower alkenyl, aryl, a heterocyclic group, or -SO₂R^{17B} (wherein R^{17B} has the same meaning as R^{12B} defined above)], -S(O)_{mb}R^{18B} (wherein mb is 0 or 1, R^{18B} represents a hydrogen atom, lower alkyl, cycloalkyl, lower alkenyl, aryl, or a heterocyclic group), or -SO₂R^{19B} (wherein R^{19B} has the same meaning as R^{12B} defined above).

The halogen, lower alkyl, lower alkenyl, lower alkadienyl, lower alkatrienyl, lower alkynyl, cycloalkyl, aryl, and heterocyclic group mentioned here, have the same meanings as the halogen (iX), lower alkyl (iv), lower alkenyl (v), lower alkadienyl (xiv), lower alkatrienyl (xv), lower alkynyl (vi), cycloalkyl (vii), aryl (viii), and heterocyclic group (x), respectively. Lower alkyl moiety of the (lower alkoxy)lower alkoxy has the same meaning as the lower alkyl (iv) defined above, and alkylene moiety of the (lower alkoxy)lower alkoxy has the same meaning as the group formed by removing one hydrogen atom from lower alkyl (iv) defined above.

Examples of production methods for the above-mentioned effective ingredients in the steroid-sulfatase inhibitors according to the present invention will now be described.

For example, the following compounds are prepared according to the respective documents: estrone-3-methylthiophosphonate, estrone-3-methylphosphonate, estrone-3-phenylphosphonothioate, and estrone-3-phenylphosphonate [Cancer Research, vol.53, p.298 (1993); Bioorganic & Medicinal Chemistry Letters, vol.3, p.313 (1993); U.S. Patent No. 5,604,215]; estrone-3-sulfamate derivatives [Journal of Medicinal Chemistry, vol.37, p.219 (1994)]; 3-desoxyestrone-3-sulfonate derivatives [Steroids, vol.58, p.106 (1993); The Journal of Steroid Biochemistry and Molecular Biology, vol.50, p.261 (1994)]; 3-desoxyestrone-3-methylsulfonate derivatives [Steroids, vol.60, p.299 (1995)]; estrone-3-amino derivatives [The Journal of Steroid Biochemistry and Molecular Biology, vol.59, p.83 (1996); U.S. Patent Nos. 5,571,933 and 5,866,603]; vitamin D₃ derivatives [The Journal of Steroid Biochemistry and Molecular Biology, vol.48, p.563 (1994)]; dehydroepiandrosterone derivatives [The Journal of Steroid Biochemistry and Molecular Biology, vol.45, p.383 (1993); Biochemistry, 36: 2586 (1997)]; estrone-3-sulfamate modifications [The Journal of Steroid Biochemistry and Molecular Biology, vol.64, p.269 (1998); WO98/24802; WO98/32763]; 17-alkylestradiol derivatives [Bioorganic & Medicinal Chemistry Letters, vol.8, p.1891 (1998); Journal of Medicinal Chemistry, vol.42, p.2280 (1999)]; 3-substituted-D-homo-1,3,5,(10)-estratriene derivatives (WO98/11124; WO99/27935); estrone modifications [WO98/42729; WO99/27936; Canadian Journal of Physiology and Pharmacology vol.76, p.99 (1998)]; 17β-(N-alkylcarbamoyl)estra-1,3,5(10)-triene-3-sulfamate and 17β-(N-alkanoylamino)estra-1,3,5(10)-triene-3-sulfamate [Steroids, vol.63, p.425 (1998); WO99/03876]; estrone modifications at the 17-position (WO99/33858); tetrahydronaphthol derivatives [Journal of Medicinal Chemistry, vol.37, p.219 (1994)]; 4-methylcoumarin-7-sulfamate [Cancer Research, vol.56, p.4950 (1996); WO97/30041] tyramine derivatives and phenol derivatives [Cancer Research, vol.57, p.702 (1997); Biochemistry, vol.36, p.2586 (1997); The Journal of Steroid Biochemistry and Molecular Biology, vol.68, p.31 (1999); U.S. Patent No. 5,567,831]; flavonoid [The Journal of Steroid Biochemistry and Molecular Biology, vol.63, p.9 (1997); WO97/32872]; 4-hydroxytamoxifen derivatives [The Journal of Steroid Biochemistry and Molecular Biology, vol.45, p.383 (1993); Bioorganic & Medicinal Chemistry Letters, vol.9, p.141 (1999)]; isoflavone derivatives [The Journal of Steroid Biochemistry and Molecular Biology, vol.69, p.227 (1999)]; and chroman derivatives (WO99/52890).

Furthermore, WO93/05064, WO01/02349, WO97/30041 WO01/36398, and WO00/43408 disclose compounds having a steroid-sulfatase inhibiting activity that can be used in the present invention, and these compounds can be prepared according to methods disclosed therein.

For hormone therapy, agents that can (a) inhibit the production of estrogen or androgen, (b) block estrogen from binding to an estrogen receptor, (c) block androgen from binding to an androgen receptor, or (d) inhibit the secretion of estrogen or luteinizing hormone may be used. Examples of these agents are antiestrogen agents, aromatase inhibitors, antiandrogen agents, LH-RH agonists, and progesterone products, and they may be used alone or in combination.

Examples of the antiestrogen agents include compositions comprising tamoxifen, ICI-182780 (trade name; Faslodex, generic name; fulvestrant), toremifene, or pharmaceutically acceptable salts thereof as active ingredients.

Examples of the aromatase inhibitors include compositions comprising amino-glutathione, anastrozole, letrozole, exemestane, vorozole, fadrozole, or pharmaceutically acceptable salts thereof as active ingredients.

Examples of the antiandrogen agents include compositions comprising flutamide, bicalutamide, nilutamide, cyproterone, or pharmaceutically acceptable salts thereof as active ingredients.

Examples of the LH-RH agonists include compositions comprising luprolide, goserelin, or pharmaceutically acceptable salts thereof as active ingredients.

Examples of the progesterone products include compositions comprising megestrol acetate, medroxyprogesterone acetate, or pharmaceutically acceptable salts thereof as active ingredients.

Examples of the chemotherapy agents include compositions comprising adriamycin, cyclophosphamide, paclitaxel, docetaxel, vinorelbine, fluorouracil, irinotecan, methotrexate, or pharmaceutically acceptable salts thereof as active ingredients.

The pharmaceutically acceptable salts of the effective ingredients that constitute the steroid-sulfatase inhibitors, agents for hormone therapy, and agents for chemotherapy are, for example, pharmaceutically acceptable acid addition salts, metal salts, ammonium salts, organic amine addition salts, and amino acid addition salts. Examples of the acid addition salts include inorganic acid salts, e.g. hydrochloride, sulfate, and phosphate; and organic acid salts, e.g. acetate, maleate, fumarate, tartrate, citrate, lactate, and succinate. Examples of the metal salts include alkali-metal salts, e.g. sodium salt and potassium salt; alkaline-earth metal salts, e.g. magnesium salts and calcium salts; aluminium salts, and zinc salts. Examples of ammonium salts include ammonium salts and tetramethylammonium salts. Examples of organic amine addition salts include addition salts of morpholine and piperidine. Examples of amino acid addition salts include addition salts of lysine, glycine, phenylalanine, aspartic acid, and glutamic acid.

Steroid-sulfatase inhibitors and agents for hormone therapy and/or agents for chemotherapy agents used in therapeutic agents and pharmaceutical compositions for hormone-dependent cancers according to the present invention may be administered alone or in combination as preparations containing their active ingredients. Particularly, a combination of two to four preparations is preferable. When the preparations are used or administered in combination, they may be used or administered together or separately at an interval.

These preparations can be manufactured by a conventional process using a pharmaceutically acceptable diluent, excipient, disintegrant, lubricant; binder, surfactant, water, saline, vegetable-oil solubilizer, isotonic agent, preservative, or antioxidant in addition to each active ingredient.

When the preparations are administered in combination, for example, a first component comprising (a) the steroid-sulfatase inhibitor and a second component comprising (b) the agent for hormone therapy and/or agent for chemotherapy are separately prepared as described above and made into a kit. By utilizing such a kit, different preparations can be administered together or separately at an interval to one subject by the same route or different routes. The second component may be further separated into several components, preferably, two or three components.

The kit is composed of at least two containers (e.g. vials, bags) and contents (i.e. the first and second components). The material and the shape of the containers are not limited, but the containers must prevent the contents, i.e. the components, from degrading due to external temperature or light during the storage, and should be made from a material that does not elute its chemical constituents. The first component and the second component are administerable dosage forms so as to be administered through different routes (e.g. tubes) or the same route. A preferable example is a kit for injection. For example, the containers of the first and second components are formed to connect to a bag containing an infusion solution so that each of the components is mixed with the infusion solution.

A method for treating hormone-dependent cancers according to the present invention can be performed similarly to the above-mentioned utilization or administration of the steroid-sulfatase inhibitor and the agent for hormone therapy and/or agent for chemotherapy used as the therapeutic agent for hormone-dependent cancers. Namely, the method can be performed by preparing the steroid-sulfatase inhibitor and the agent for hormone therapy and/or agent for chemotherapy so as to contain their active ingredients and by administering alone or in combination, preferably, in a combination of two to four preparations. When the preparations are administered in combination, they may be administered together or separately at an interval and may also be administered in the form of a kit as described above.

The efficiency of hormone-dependent cancer treatment by the combined administration of a steroid-sulfatase inhibitor and an agent for hormone therapy will be explained in detail by referring to Experimental Examples.

### Experimental Example 1: Proliferation inhibition of breast cancer cells using combination of antiestrogen agent and steroid-sulfatase inhibitor

(1) MCS-2 cell in which human steroid-sulfatase is excessively expressed, was established from human breast cancer cell (MCF-7). Inhibition of the MCS-2 cell proliferation by an antiestrogen agent alone or a steroid-sulfatase inhibitor alone was compared with that by a combination of the steroid-sulfatase inhibitor and the antiestrogen agent. ICI-182780 was used as the antiestrogen agent, and compound 1 was used as the steroid-sulfatase inhibitor.

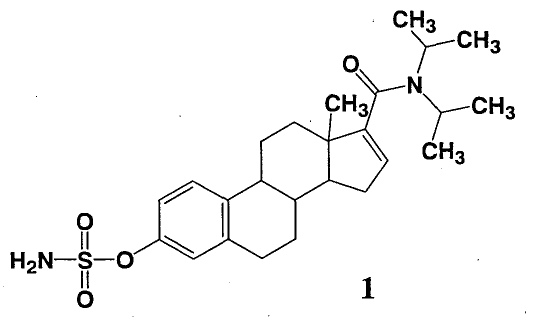

The MCS-2 cells were subcultured in a Phenol Red-free Eagle's minimum essential medium [PR(-)MEM; Nissui Pharmaceutical Co., Ltd.: referred to as medium A hereinafter] containing 5% bovine fetal serum (HyClone Laboratories Inc.) treated with dextran-charcoal, 1 mmol/L sodium pyruvate (Wako Pure Chemical Industries, Ltd.), 1% nonessential amino acid (NEAA; Dainippon Pharmaceutical Co., Ltd.), 2 mmol/L L-glutamine (GIBCO BRL), and 0.11% sodium hydrogencarbonate solution (ICN Biomedicals Inc.).

A dimethylsulfoxide (DMSO; Kanto Kagaku) solution containing 10 mmol/L estrone sulfate (Sigma Corp.) was diluted with medium A to a final concentration of 10⁻⁸ mol/L (medium B).

MCS-2 cells were diluted with the medium B containing estrone sulfate (final concentration: 10⁻⁸ mol/L) to a concentration of 2.5 × 10⁴ cells/mL and inoculated in a 96-well microtiter plate (NUNC) in an amount of 100 µL/well. The plate was incubated in an incubator set at 37°C and a humidity of 95% or more in a 5%-CO₂ atmosphere for 24 hours, and then the medium was replaced with fresh estrone sulfate-containing medium B or fresh estrone sulfate-free medium A. To each well in which the medium was replaced with fresh estrone sulfate-containing medium B, test compound [(i) antiestrogen agent alone, (ii) a steroid-sulfatase inhibitor alone, or (iii) a combination of the antiestrogen agent and the steroid-sulfatase inhibitor: these agents were sequentially diluted with medium A] was added. To each well in which the medium was replaced with fresh estrone sulfate-free medium A, the agent was not added [(iv) agent-free]. The plate was incubated in an incubator set at 37°C and a humidity of 95% or more in a 5%-CO₂ atmosphere for 168 hours. After the incubation, the supernatant was carefully removed such that all the cells retained. Then, an MTT solution, which was prepared by dissolving 3-(4,5-dimethylthiazol-2-yl)-2,5-diphenyltetrazolium bromide (Sigma Corp.) in estrone sulfate-free medium A into a final concentration of 0.5 mg/mL, was added to each well in an amount of 50 µL/well. The plate was incubated in an incubator set at 37°C in a 5%-CO₂ atmosphere for 4 hours. After the MTT solution was removed, 0.1 mL of DMSO was added to each well. The plate was stirred with a plate mixer (Micro Mixer Model MX-4; Sanko Junyaku Co., Ltd.), and the formed formazan was eluted to measure the difference in absorbance at 550 nm and 630 nm with a plate reader (Spectra MAX 250; Wako Pure Chemical Industries, Ltd.). The results of inhibition of MCS-2 cell proliferation were indicated by a relative value of the number of MCS-2 cells in each condition to that in the agent-free condition, and are shown as a relative value (%) of each absorbance (MTT assay).

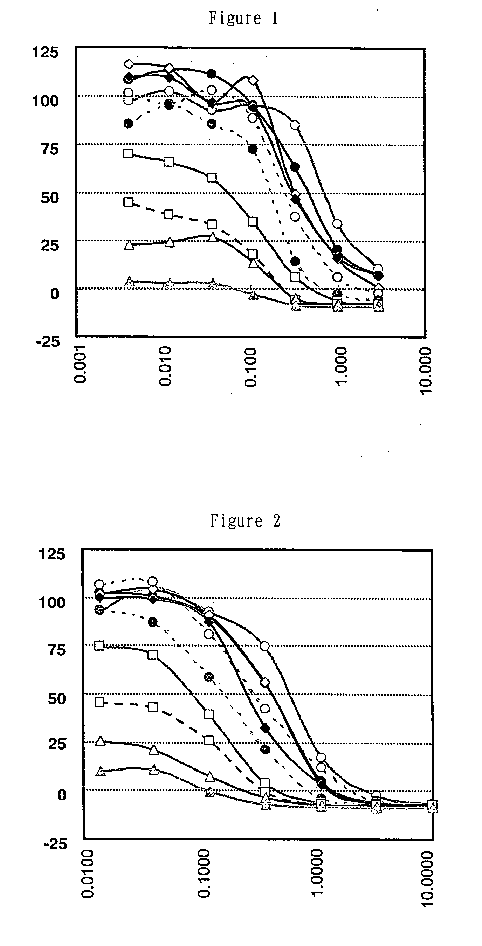

Fig. 1 shows inhibition curves on the MCS-2 cell proliferation under constant ICI-182780 concentrations while the concentration of Compound 1 was varied. Fig. 2 shows inhibition curves on the MCS-2 cell proliferation under constant Compound 1 concentrations while the concentration of ICI-182780 was varied.

As shown in Fig. 1, the inhibition of MCS-2 cell proliferation by Compound 1 was facilitated by the addition of ICI-182780 at a concentration of 0.234 nmol/L or more as compared with the results of the case ICI-182780 was not added.

As shown in Fig. 2, the inhibition of MCS-2 cell proliferation by ICI-182780 was facilitated by the addition of Compound 1 at a concentration of 0.140 nmol/L or more as compared with the results of the case Compound 1 was not added.

(2) In order to evaluate the efficacy of the combination treatment with the antiestrogen agent and the steroid-sulfatase inhibitor, an isobologram was constructed from the inhibition curves on the MCS-2 cell proliferation shown in Figs. 1 and 2 according to International Journal of Radiation Oncology Biology Physics, page 85 (1979) and page 1145 (1979).

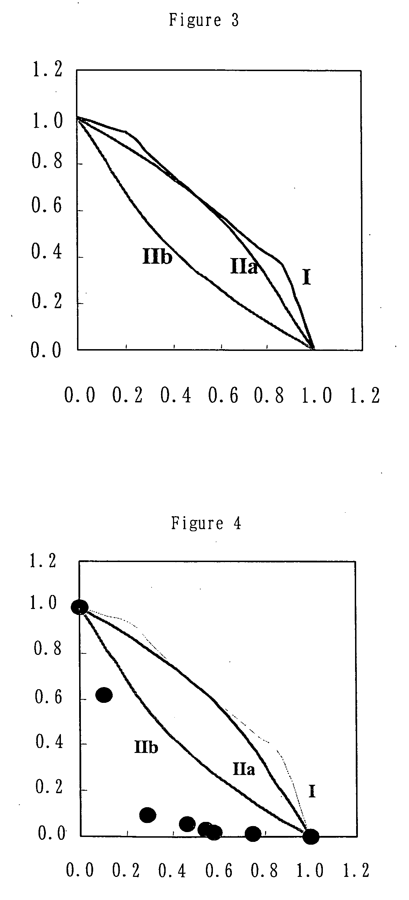

When the antiestrogen agent or the steroid-sulfatase inhibitor was used alone, the concentrations of each required to inhibit the proliferation of MCS-2 cells, i.e. the concentrations required to inhibit 50% of the proliferation (IC₅₀ value), inhibit 45% of the proliferation (IC₄₅ value), inhibit 40% of the proliferation (IC₄₀ value), inhibit 35% of the proliferation (IC₃₅ value), inhibit 30% of the proliferation (IC₃₀ value), inhibit 25% of the proliferation (IC₂₅ value), inhibit 20% of the proliferation (IC₂₀ value), inhibit 15% of the proliferation (IC₁₅ value), inhibit 10% of the proliferation (IC₁₀ value), and inhibit 5% of the proliferation (IC₅ value), were calculated from the inhibition curves on the MCS-2 cell proliferation shown in Figs. 1 and 2 in the above-mentioned (1) with measurement software (the equation used in Soft Max Pro) equipped with a plate reader. An isobologram for IC₅₀ values was constructed using these IC₅ to IC₅₀ values according to the above-mentioned paper. The isobologram is shown in Fig. 3.

(3) In order to evaluate the efficacy of the combination treatment, each concentration of the agents showing IC₅₀ values in combination was plotted on the isobologram shown in Fig. 3 according to a method described in International Journal of Radiation Oncology Biology Physics, page 85 (1979) and page 1145 (1979).

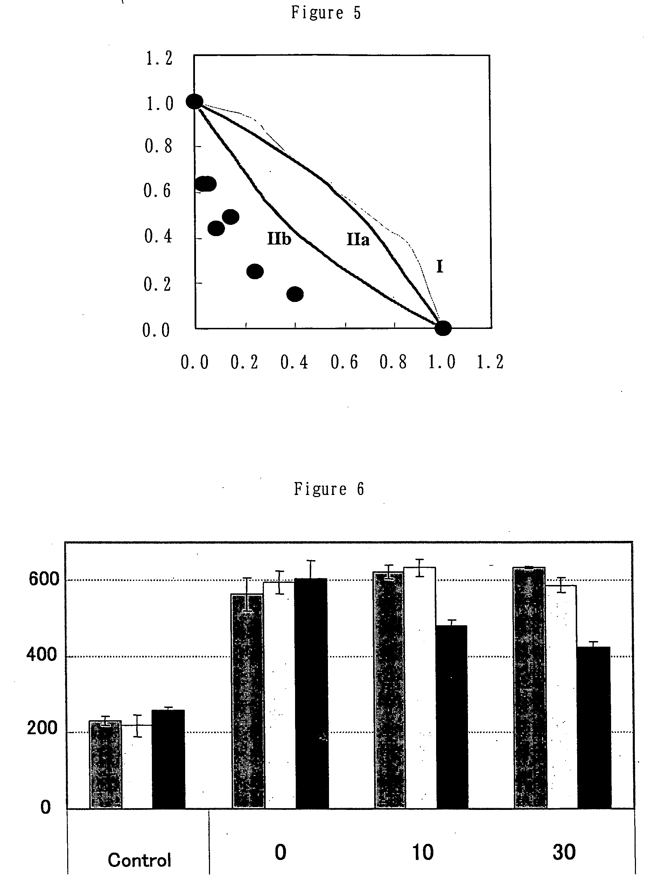

IC₅₀ values under the conditions where ICI-182780 concentrations were constant, i.e. concentrations of Compound 1 when 50% of the proliferation of MCS-2 cells was inhibited, were plotted on the isobologram (Fig. 3) constructed in the above-mentioned (2). The results are shown in Fig. 4. IC₅₀ values under the conditions where Compound 1 concentrations were constant, i.e. concentrations of ICI-182780 when 50% of the proliferation of MCS-2 cells was inhibited, were plotted on the isobologram (Fig. 3) constructed in the above-mentioned (2). The results are shown in Fig. 5.

When the plots lay below a line of mode I on the isobologram, it is determined that the combination treatment had an additive effect. When the plots lay below a line of mode IIa, it is determined that the combination treatment further had a supra-additive effect.

As shown in Fig. 4, when ICI-182780 was used at constant concentrations, the addition of Compound 1 supra-additively inhibited the cell proliferation.

As shown in Fig. 5, when Compound 1 was used at constant concentrations, the addition of ICI-182780 supra-additively inhibited the cell proliferation.

### Experimental Example 2: Proliferation inhibition of breast cancer cells using combination of aromatase inhibitor and steroid-sulfatase inhibitor

Proliferation inhibition of breast cancer cell line (MCS-2) excessively expressing a human steroid-sulfatase by using an aromatase inhibitor alone or a steroid-sulfatase inhibitor alone was compared with that using a combination of the steroid-sulfatase inhibitor and the aromatase inhibitor. Vorozole was used as the aromatase inhibitor, and Compound 1 was used as the steroid-sulfatase inhibitor.

Medium C containing 10⁻⁸ mol/L estrone sulfate (final concentration) and 10⁻⁷ mol/L testosterone (final concentration) was prepared by diluting a DMSO solution containing 10 mmol/L estrone sulfate (Sigma Corp.) and a DMSO solution containing 10 mmol/L testosterone (Sigma Corp.) with medium A used in Experimental Example 1.

MCS-2 cells were diluted with medium A to 2.5 × 10⁴ cells/mL, and then were inoculated in a 24-well microtiter plate (NUNC) at an amount of 100 µL/well. The plate was incubated in an incubator set at 37°C and a humidity of 95% or more in a 5%-CO₂ atmosphere for 24 hours, and then the medium was replaced with fresh medium C or fresh medium A. Medium C contained estrone sulfate at a final concentration of 10⁻⁸ mol/L and testosterone at a final concentration of 10⁻⁷ mol/L. Medium A contained neither estrone sulfate nor testosterone. To each well in which the medium was replaced with medium C, test compound diluted with a medium A [(i) an aromatase inhibitor alone, (ii) a steroid-sulfatase inhibitor alone, or (iii) a combination of the aromatase inhibitor and the steroid-sulfatase inhibitor] was added, or was not added [(iv) agent-free]. The plate was incubated in an incubator set at 37°C and a humidity of 95% or more in a 5%-CO₂ atmosphere for 168 hours. The test compound were not added to the wells in which the medium was replaced with medium A (which contained neither estrone sulfate nor testosterone). These wells were incubated under the same conditions as above and used as controls. After the incubation, the supernatant was carefully removed such that all the cells retained. Then, the wells were carefully rinsed with a phosphate buffer (GIBCO) such that all the cells retained. A solution of 0.25% trypsin (GIBCO) and an aqueous solution of 0.02% ethylenediaminetetraacetic acid (EDTA, Wako Pure Chemical Industries, Ltd.) were added to the wells to thoroughly suspend the cells. The number of cells in each well was counted with a microcell counter (Sysmex).

Fig. 6 shows the numbers of MCS-2 cells in the presence of estrone sulfate and testosterone, when an aromatase inhibitor (vorozole), a steroid-sulfatase inhibitor (Compound 1), or both vorozole and Compound 1 were added (N = 3 each).

When Compound 1 was not added, inhibition of the cell proliferation was very low regardless of an increase in the concentration of vorozole. That is, the inhibition of the proliferation was able to be observed at a high concentration of 100 nmol/L. On the contrary, when Compound 1 was added, significant inhibition of the cell proliferation was observed.

The therapeutic agents and pharmaceutical compositions for the treatment of hormone-dependent cancers according to the present invention, which are prepared so as to contain active ingredients from both steroid-sulfatase inhibitors and agents for hormone therapy and/or agents for chemotherapy, can be used, administered, or manufactured in the form of a single preparation or a combination of some preparations. These therapeutic agents in unit dose form are preferable for oral or parenteral (e.g. injection) administration. When the therapeutic agents are used or administered in combination, they may be used or administered together or separately at an interval.

These preparations may contain a pharmaceutically acceptable diluent, excipient, disintegrant, lubricant, binder, surfactant, water, saline, vegetable-oil solubilizer, isotonic agent, preservative, or antioxidant in addition to the effective ingredients, and can be manufactured by a conventional process.

In the preparation of tablets, an excipient, e.g. lactose, a disintegrant, e.g. starch, a lubricant, e.g. magnesium stearate, a binder, e.g. hydroxypropyl cellulose, a surfactant, e.g. fatty acid ester, a plasticizer, e.g. glycerin, and the like may be used according to a conventional process.

In the preparation of injections, water, saline, a vegetable-oil, a solvent, a solubilizer, an isotonic agent, a preservative, an antioxidant, and the like may be used according to a conventional process.

When compound (I), (IA), (IB), and pharmaceutically acceptable salts thereof are used for the above-mentioned purposes, they may be administered orally or parenterally such as injections. An effective dose and frequency of administration depend on the administration form and subject's age, weight, and symptoms. In general, 0.01 to 20 mg/kg/day is preferably administered.

### Brief Description of the Drawings

Fig. 1 shows the results of an MTT assay showing the inhibition of MCS-2 cell proliferation when the concentrations of ICI-182780 were constant in the range from 0 to 1.801 nmol/L and Compound 1 was sequentially diluted (1.5-fold for each dilution) from 3.000 nmol/L to 0.004 nmol/L (N = 3 each). The vertical axis of the graph represents a relative value of the number of MCS-2 cells in each condition to that in the agent-free condition, which is shown as a relative value (%) of each absorbance. On the horizontal axis of the graph, the amounts of Compound 1 (nmol/L) are shown. Plots on the graph represent concentrations (nmol/L) of ICI-182780.
-○-: ICI-182780 free
-●-: ICI-182780 at a concentration of 0.030 nmol/L
-◇ -: ICI-182780 at a concentration of 0.051 nmol/L
-◆-: ICI-182780 at a concentration of 0.084 nmol/L
--○--: ICI-182780 at a concentration of 0.140 nmol/L
--●--: ICI-182780 at a concentration of 0.234 nmol/L
-□-: ICI-182780 at a concentration of 0.390 nmol/L
--□--: ICI-182780 at a concentration of 0.649 nmol/L
-Δ-: ICI-182780 at a concentration of 1.081 nmol/L
-▲-: ICI-182780 at a concentration of 1.801 nmol/L

Fig. 2 shows the results of an MTT assay showing the inhibition of MCS-2 cell proliferation when the concentrations of Compound 1 were constant in the range from 0 to 1.081 nmol/L and ICI-185780 was sequentially diluted (1.5-fold for each dilution) from 10.000 nmol/L to 0.014 nmol/L (N = 3 each). The vertical axis of the graph represents a relative value of the number of MCS-2 cells in each condition to that in the agent-free condition, which is shown as a relative value (%) of each absorbance. On the horizontal axis of the graph, the amounts of ICI-182780 (nmol/L) are shown. Plots on the graph represent concentrations (nmol/L) of Compound 1 shown below.
-○-: Compound 1 free
-●-: Compound 1 at a concentration of 0.018 nmol/L
-◇ -: Compound 1 at a concentration of 0.030 nmol/L
-◆-: Compound 1 at a concentration of 0.051 nmol/L
--○--: Compound 1 at a concentration of 0.084 nmol/L
--●--: Compound 1 at a concentration of 0.140 nmol/L
-□-: Compound 1 at a concentration of 0.234 nmol/L
--□--: Compound 1 at a concentration of 0.389 nmol/L
-Δ-: Compound 1 at a concentration of 0.649 nmol/L
-▲-: Compound 1 at a concentration of 1.081 nmol/L

Fig. 3 shows an isobologram for the IC₅₀ value constructed from an IC₅₀ value, IC₄₅ value, IC₄₀ value, IC₃₅ value, IC₃₀ value, IC₂₅ value, IC₂₀ value, IC₁₅ value, IC₁₀ value, and IC₅ value calculated from the inhibition curves on the MCS-2 cell proliferation shown in Figs. 1 and 2 for both ICI-182780 alone and Compound 1 alone. The vertical axis of the graph represents a fraction of IC₅₀ of ICI-182780, and the horizontal axis represents that of Compound 1.

Fig. 4 shows the concentrations (IC₅₀ values) calculated from the inhibition curves shown in Fig. 1 when Compound 1 inhibited 50% of the proliferation of MCS-2 cells under the conditions where the concentrations of ICI-182780 were constant in the range from 0.030 nmol/L to 1.801 nmol/L and Compound 1 was sequentially diluted (1.5-fold for each dilution) from 3.000 nmol/L to 0.004 nmol/L. The concentrations of Compound 1 are plotted with symbol ● on the isobologram (Fig. 3).

Fig. 5 shows the concentrations (IC₅₀ values) calculated from the inhibition curves shown in Fig. 2 when ICI-182780 inhibited 50% of the proliferation of MCS-2 cells under the conditions where the concentrations of Compound 1 were constant in the range from 0.018 nmol/L to 1.081 nmol/L and ICI-182780 was sequentially diluted (1.5-fold for each dilution) from 10.000 nmol/L to 0.014 nmol/L. The concentrations of ICI-182780 are plotted with symbol ● on the isobologram (Fig. 3).

Fig. 6 shows the inhibition of MCS-2 cell proliferation when a combination of vorozole and Compound 1 was used in the presence of estrone sulfate and testosterone. The vertical axis of the graph represents the number of MCS-2 cells (× 10³ cells/mL), and the horizontal axis represents control and vorozole concentration (nmol/L). The three bars show the results when Compound 1 was added at a concentration of, from the left, 0 nmol/L, 3.0 nmol/L, and 10.0 nmol/L.

### Best Mode For Carrying Out the Invention

The formulation examples will be illustrated below, however these examples are never intended to limit the scope of the present invention.

### Formulation Example 1 (Tablet)

Tablets having the following composition are prepared according to a conventional procedure.

| | |
|---|---|
| Compound 1 | 5 mg |
| Lactose | 60 mg |
| Potato starch | 30 mg |
| Polyvinylalcohol | 2 mg |
| Magnesium stearate | 1 mg |
| Tar pigment | small amount |

### Formulation Example 2 (Tablet)

Tablets having the following composition are prepared according to a conventional procedure.

| | |
|---|---|
| Compound 1 | 5 mg |
| Tamoxifen | 10 mg |
| Lactose | 60 mg |
| Potato starch | 30 mg |
| Polyvinylalcohol | 2 mg |
| Magnesium stearate | 1 mg |
| Tar pigment | small amount |

### Preparation Example 3 (Injection)

An injection having the following composition is prepared according to a conventional procedure.

| | |
|---|---|
| Compound 1 | 2 mg |
| D-mannitol | 10 mg |
| Hydrocholoric acid solution | proper amount |
| Sodium hydroxide solution | proper amount |
| Injectable distilled water | proper amount |

### Industrial Applicability

According to the present invention, a therapeutic agent for a hormone-dependent cancer, which comprises (a) a steroid-sulfatase inhibitor and (b) an agent for hormone therapy and/or an agent for chemotherapy, and the like are provided. The above agent shows more excellent activity in treating a hormone-dependent cancer than a steroid-sulfatase alone or an agent for hormone therapy and/or an agent for chemotherapy alone.

## Claims

1. A therapeutic agent for a hormone-dependent cancer, which comprises (a) a steroid-sulfatase inhibitor and (b) an agent for hormone therapy and/or an agent for chemotherapy, which may be administered together or separately at an interval.

2. A method for treating a hormone-dependent cancer, which comprises administering (a) a steroid-sulfatase inhibitor and (b) an agent for hormone therapy and/or an agent for chemotherapy together or separately at an interval.

3. A steroid-sulfatase inhibitor which is used in combination with an agent for hormone therapy and/or an agent for chemotherapy, and which is administered together therewith or separately therefrom at an interval.

4. A kit for treating a hormone-dependent cancer, which comprises a first component comprising (a) a steroid-sulfatase inhibitor and a second component comprising (b) an agent for hormone therapy and/or an agent for chemotherapy.

5. A pharmaceutical composition, which comprises (a) a steroid-sulfatase inhibitor and (b) an agent for hormone therapy and/or an agent for chemotherapy.

6. Use of (a) a steroid-sulfatase inhibitor and (b) an agent for hormone therapy and/or an agent for chemotherapy for the manufacture of a therapeutic agent for a hormone-dependent cancer.

7. The therapeutic agent for a hormone-dependent cancer according to Claim 1, wherein the steroid-sulfatase inhibitor is a composition comprising, as an active ingredient, a compound represented by Formula (I) or a pharmaceutically acceptable salt thereof: 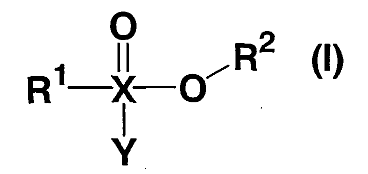 [wherein X represents a phosphorus atom or a sulfur atom, and when X is a phosphorus atom, Y is hydroxy, and when X is a sulfur atom, Y is oxo; R¹ represents a hydrogen atom, substituted or unsubstituted lower alkyl, substituted or unsubstituted cycloalkyl, substituted or unsubstituted lower alkenyl, substituted or unsubstituted aryl, or -NR³R⁴ (wherein R³ and R⁴ may be the same or different and each represents a hydrogen atom, substituted or unsubstituted lower alkyl, substituted or unsubstituted cycloalkyl, substituted or unsubstituted lower alkenyl, or substituted or unsubstituted aryl, or R³ and R⁴ are combined together with the adjacent nitrogen atom thereto to form a substituted or unsubstituted heterocyclic group); and -O-R² represents a monocyclic alcohol residue or a polycyclic alcohol residue].

8. The method for treating a hormone-dependent cancer according to Claim 2, wherein the steroid-sulfatase inhibitor is a composition comprising, as an active ingredient, the compound represented by Formula (I) described in Claim 7 or a pharmaceutically acceptable salt thereof.

9. The steroid-sulfatase inhibitor according to Claim 3, wherein the steroid-sulfatase inhibitor is a composition comprising, as an active ingredient, the compound represented by Formula (I) described in Claim 7 or a pharmaceutically acceptable salt thereof.

10. The kit for treating according to Claim 4, wherein the steroid-sulfatase inhibitor is a composition comprising, as an active ingredient, the compound represented by Formula (I) described in Claim 7 or a pharmaceutically acceptable salt thereof.

11. The pharmaceutical composition according to Claim 5, wherein the steroid-sulfatase inhibitor is a composition comprising, as an active ingredient, the compound represented by Formula (I) described in Claim 7 or a pharmaceutically acceptable salt thereof.

12. The use of (a) a steroid-sulfatase inhibitor and (b) an agent for hormone therapy and/or an agent for chemotherapy according to Claim 6, wherein the steroid-sulfatase inhibitor is a composition comprising, as an active ingredient, the compound represented by Formula (I) described in Claim 7 or a pharmaceutically acceptable salt thereof.

13. The therapeutic agent for a hormone-dependent cancer according to Claim 1, wherein the steroid-sulfatase inhibitor is a composition comprising, as an active ingredient, a compound represented by Formula (IA) or a pharmaceutically acceptable salt thereof: 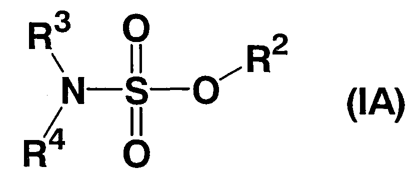 (wherein -O-R², R³, and R⁴ have the same meanings as defined above, respectively).

14. The method for treating a hormone-dependent cancer according to Claim 2, wherein the steroid-sulfatase inhibitor is a composition comprising, as an active ingredient, the compound represented by Formula (IA) described in Claim 13 or a pharmaceutically acceptable salt thereof.

15. The steroid-sulfatase inhibitor according to Claim 3, wherein the steroid-sulfatase inhibitor is a composition comprising, as an active ingredient, the compound represented by Formula (IA) described in Claim 13 or a pharmaceutically acceptable salt thereof.

16. The kit for treating according to Claim 4, wherein the steroid-sulfatase inhibitor is a composition comprising, as an active ingredient, the compound represented by Formula (IA) described in Claim 13 or a pharmaceutically acceptable salt thereof.

17. The pharmaceutical composition according to Claim 5, wherein the steroid-sulfatase inhibitor is a composition comprising, as an active ingredient, the compound represented by Formula (IA) described in Claim 13 or a pharmaceutically acceptable salt thereof.

18. The use of (a) a steroid-sulfatase inhibitor and (b) an agent for hormone therapy and/or an agent for chemotherapy according to Claim 6, wherein the steroid-sulfatase inhibitor is a composition comprising, as an active ingredient, the compound represented by Formula (IA) described in Claim 13 or a pharmaceutically acceptable salt thereof.

19. The therapeutic agent for a hormone-dependent cancer according to Claim 1, wherein the steroid-sulfatase inhibitor is a composition comprising, as an active ingredient, a compound represented by Formula (IB) or a pharmaceutically acceptable salt thereof: 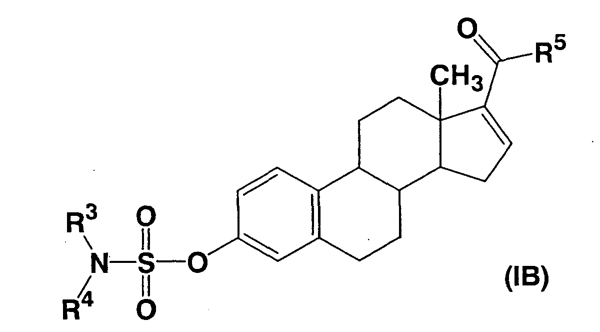 [wherein R³ and R⁴ have the same meanings as defined above, respectively; R⁵ represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, -NR⁶R⁷ (wherein R⁶ and R⁷ may be the same or different and each represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), -OR⁸ (wherein R⁸ represents a hydrogen atom, substituted or unsubstituted lower alkyl, substituted or unsubstituted cycloalkyl, substituted or unsubstituted lower alkenyl, substituted or unsubstituted aryl, or a substituted or unsubstituted heterocyclic group), or -SR^{8A} (wherein R^{8A} has the same meaning as R⁸ defined above)].

20. The method for treating a hormone-dependent cancer according to Claim 2, wherein the steroid-sulfatase inhibitor is a composition comprising, as an active ingredient, the compound represented by Formula (IB) described in Claim 19 or a pharmaceutically acceptable salt thereof.

21. The steroid-sulfatase inhibitor according to Claim 3, wherein the steroid-sulfatase inhibitor is a composition comprising, as an active ingredient, the compound represented by Formula (IB) described in Claim 19 or a pharmaceutically acceptable salt thereof.

22. The kit for treating according to Claim 4, wherein the steroid-sulfatase inhibitor is a composition comprising, as an active ingredient, the compound represented by Formula (IB) described in Claim 19 or a pharmaceutically acceptable salt thereof.

23. The pharmaceutical composition according to Claim 5, wherein the steroid-sulfatase inhibitor is a composition comprising, as an active ingredient, the compound represented by Formula (IB) described in Claim 19 or a pharmaceutically acceptable salt thereof.

24. The use of (a) a steroid-sulfatase inhibitor and (b) an agent for hormone therapy and/or an agent for chemotherapy according to Claim 6, wherein the steroid-sulfatase inhibitor is a composition comprising, as an active ingredient, the compound represented by Formula (IB) described in Claim 19 or a pharmaceutically acceptable salt thereof.

25. The therapeutic agent for a hormone-dependent cancer according to Claim 1, 7, 13, or 19, wherein the agent for hormone therapy is one or more selected from the group consisting of an antiestrogen agent, an aromatase inhibitor, an antiandrogen agent, a preparation comprising progesterone, and a preparation comprising a luteinizing hormone-releasing hormone (LH-RH) agonist.

26. The method for treating a hormone-dependent cancer according to Claim 2, 8, 14, or 20, wherein the agent for hormone therapy is one or more selected from the group consisting of an antiestrogen agent, an aromatase inhibitor, an antiandrogen agent, a preparation comprising progesterone, and a preparation comprising a LH-RH agonist.

27. The steroid-sulfatase inhibitor according to Claim 3, 9, 15, or 21, wherein the agent for hormone therapy is one or more selected from the group consisting of an antiestrogen agent, an aromatase inhibitor, an antiandrogen agent, a preparation comprising progesterone, and a preparation comprising a LH-RH agonist.

28. The kit for treating according to Claim 4, 10, 16, or 22, wherein the agent for hormone therapy is one or more selected from the group consisting of an antiestrogen agent, an aromatase inhibitor, an antiandrogen agent, a preparation comprising progesterone, and a preparation comprising a LH-RH agonist.

29. The pharmaceutical composition according to Claim 5, 11, 17, or 23, wherein the agent for hormone therapy is one or more selected from the group consisting of an antiestrogen agent, an aromatase inhibitor, an antiandrogen agent, a preparation comprising progesterone, and a preparation comprising a LH-RH agonist.

30. The use of (a) a steroid-sulfatase inhibitor and (b) an agent for hormone therapy and/or an agent for chemotherapy according to Claim 6, 12, 18, or 24, wherein the agent for hormone therapy is one or more selected from the group consisting of an antiestrogen agent, an aromatase inhibitor, an antiandrogen agent, a preparation comprising progesterone, and a preparation comprising a LH-RH agonist.

31. The therapeutic agent for a hormone-dependent cancer according to Claim 1, 7, 13, or 19, wherein the agent for hormone therapy is an antiestrogen agent and/or an aromatase inhibitor.

32. The method for treating a hormone-dependent cancer according to Claim 2, 8, 14, or 20, wherein the agent for hormone therapy is an antiestrogen agent and/or an aromatase inhibitor.

33. The steroid-sulfatase inhibitor according to Claim 3, 9, 15, or 21, wherein the agent for hormone therapy is an antiestrogen agent and/or an aromatase inhibitor.

34. The kit for treating according to Claim 4, 10, 16, or 22, wherein the agent for hormone therapy is an antiestrogen agent and/or an aromatase inhibitor.

35. The pharmaceutical composition according to Claim 5, 11, 17, or 23, wherein the agent for hormone therapy is an antiestrogen agent and/or an aromatase inhibitor.

36. The use of (a) a steroid-sulfatase inhibitor and (b) an agent for hormone therapy and/or an agent for chemotherapy according to Claim 6, 12, 18, or 24, wherein the agent for hormone therapy is an antiestrogen agent and/or an aromatase inhibitor.
